# EUROPEAN PATENT APPLICATION

(11) **EP 1 726 321 A2**
(43) Date of publication of application: **29.11.2006**
(21) Application number: 05022738.8
(22) Date of filing: 19.10.2005
(51) Int. Cl.: A61M 5/50, A61M 5/32, A61M 5/315

(54) **Safety hypodermic syringe**

(30) Priority: 26.05.2005 CN 200510074383; 14.09.2005 CN 200510102487
(71) Applicant: Chen, Chang-Tzu, Taipei (TW)
(72) Inventor: Chen, Chang-Tzu, Taipei (TW)
(74) Representative: Helms, Joachim

(57) **Abstract**

A safety hypodermic syringe having a thin breakable wall portion (16) at the front wall (15) of the barrel (1) thereof that is breakable with the plunger (3) of the syringe when the user forcing the barbed front tip (34) of the plunger into engagement with the tubular front neck (14) of the barrel after the service of the syringe, for enabling the plunger to carry the separated tubular front neck and the attached needle assembly (2) to the inside of the barrel after the service of the syringe to prevent contamination.

## Description

### BACKGROUND OF THE INVENTION

### 1. Field of the Invention:

The present invention relates to a safety hypodermic syringe and more particularly, to a self-destructive safety hypodermic syringe that can be destructed to keep the needle assembly firmly received inside the barrel after the service of the syringe.

### 2. Description of the Related Art:

A hypodermic syringe is an important medical instrument adapted for use to inject or withdraw liquid medicines. However, improper use of a hypodermic syringe or improper disposal of a used hypodermic syringe may cause a contamination. In order to prevent contamination after use, safety hypodermic syringes are developed. A safety hypodermic syringe is known comprising a needle assembly, a medicine barrel, a plunger, and an outer barrel. The needle assembly is fastened to the front side of the medicine barrel. The plunger is axially movably coupled to the medicine barrel and mounted with the medicine barrel inside the outer barrel. After the service of the hypodermic syringe, the plunger is pulled backwards to carry the needle cannula or the needle assembly backwards to the inside of the medicine barrel. This design of safety hypodermic syringe is functional, however it has a complicated structure and comprised of a big number of parts, resulting in a high manufacturing cost.

Further, according to conventional designs, a residual amount of the applied liquid medicine will be left in the tubular front neck of the barrel and the hub of the needle assembly after the stopper has been pushed to the front limit position and stopped at the front wall of the barrel.

Therefore, it is desirable to provide a safety hypodermic syringe that eliminates the aforesaid drawbacks.

### SUMMARY OF THE INVENTION

The present invention has been accomplished under the circumstances in view. It is the main object of the present invention to provide a safety hypodermic syringe, which is self-destructive, preventing reuse of the syringe. It is another object of the present invention to provide a safety hypodermic syringe, which expels liquid medicine completely out of the syringe during the injection, preventing residual liquid medicine in the barrel. It is still another object of the present invention to provide a safety hypodermic syringe, which keeps the needle assembly firmly secured to the inside of the barrel, preventing possible contamination.

According to one embodiment of the present invention, the safety hypodermic syringe comprises a barrel, the barrel comprising a cylindrical body, the cylindrical body having a front wall, and a tubular front neck forwardly extending from the front wall, the front wall of the cylindrical body having a thin wall portion that forms at least one breakable wall portion through which the front wall of the cylindrical body is breakable for enabling the tubular front neck to be separated from the cylindrical body; a needle assembly, the needle assembly comprising a hub fastened to the tubular front neck of the barrel and defining a fluid passage, and a needle cannula fastened to the hub; a plunger axially movably inserted into the cylindrical body of the barrel, the plunger comprising a shank, a head formed integral with a front end of the shank, and a barbed tip forwardly extending from the head; and a flexible stopper mounted on the head of the plunger and peripherally maintained in close contact with the periphery of the cylindrical body of the barrel and axially movable with the plunger relative to the barrel, the flexible stopper having a front center through hole through which the barbed tip of the plunger extends to the outside of the flexible stopper for engaging into the fluid passage of the tubular front neck of the barrel when the plunger is pushed forward and forced against the front wall of the cylindrical body of the barrel to break the at least one breakable wall portion and to separate the tubular front neck from the cylindrical body for enabling the needle assembly to be carried with the separated tubular front neck by the plunger into the inside of the barrel after the service of the safety hypodermic syringe.

According to another embodiment of the present invention, the safety hypodermic syringe comprises a barrel, the barrel comprising a cylindrical body, the cylindrical body having a front wall, and a tubular front neck forwardly extending from the front wall, the front wall of the cylindrical body having a thin wall portion that forms at least one breakable wall portion through which the front wall of the cylindrical body is breakable for enabling the tubular front neck to be separated from the cylindrical body, the tubular front neck having an inside annular flange; a needle assembly, the needle assembly comprising a hub fastened to the tubular front neck of the barrel and defining a fluid passage, and a needle cannula fastened to the hub; a plunger axially movably inserted into the cylindrical body of the barrel, the plunger comprising a shank and a head formed integral with a front end of the shank; and a flexible stopper mounted on a front side of the head of the plunger and peripherally maintained in close contact with the periphery of the cylindrical body of the barrel and axially movable with the plunger relative to the barrel, the flexible stopper having a thin wall portion and a thick wall portion disposed at two opposite lateral sides. When the plunger is pushed forwards and forced against the front wall of the cylindrical body of the barrel, the at least one breakable wall portion is broken to separate the tubular front neck of the barrel from the cylindrical body for enabling the needle assembly to be carried with the separated tubular front neck by the plunger into the inside of the barrel after the service of the safety hypodermic syringe, and at the same time the stopper is deformed to tilt the needle assembly inside the cylindrical body.

According to still another embodiment of the present invention, the safety hypodermic syringe comprises a barrel, the barrel comprising a cylindrical body, the cylindrical body having a front wall, and a tubular front neck forwardly extending from the front wall of the cylindrical body and defining a fluid passage; a needle assembly, the needle assembly comprising a hub fastened to the tubular front neck of the barrel and defining a fluid passage, and a needle cannula fastened to the hub; a plunger axially movably inserted into the cylindrical body of the barrel, the plunger comprising a shank, a head formed integral with a front end of the shank, and a front tip forwardly extending from the head; and a flexible stopper mounted on the head of the plunger over the front tip and peripherally maintained in close contact with the periphery of the cylindrical body of the barrel and axially movable with the plunger relative to the barrel, the flexible stopper having a front center through hole through which the front tip of the plunger is moved into the inside of the tubular front neck of the barrel to expel residual liquid medicine out of the barrel when the stopper is forced by the plunger against the front wall of the cylindrical body of the barrel during the application of the safety hypodermic syringe.

### BRIEF DESCRIPTION OF THE DRAWINGS

FIG. 1 is an exploded view of a safety hypodermic syringe according to a first embodiment of the present invention.
FIG. 2 is an elevational assembly view, partially cutaway of the safety hypodermic syringe according to the first embodiment of the present invention.
FIG. 3 is a sectional view of the present invention showing a status of the safety hypodermic syringe after the service.
FIG. 4 corresponds to FIG. 3, showing the stopper forced against the front wall of the cylindrical body of the barrel, the breakable wall portion broken.
FIG. 5 corresponds to FIG. 4, showing the needle assembly and the separated tubular front neck moved into the inside of the barrel.
FIG. 6 corresponds to FIG. 5, showing the shank of the plunger removed from the head, the head with the stopper and the needle assembly secured to the inside of the barrel.
FIG. 7 is an exploded view of a safety hypodermic syringe according to a second embodiment of the present invention.
FIG. 8 is an elevational view of the stopper for the safety hypodermic syringe according to the second embodiment of the present invention.
FIG. 9 is a top sectional plain view of the stopper for the safety hypodermic syringe according to the second embodiment of the present invention.
FIG. 10 is a front sectional view of the stopper for the safety hypodermic syringe according to the second embodiment of the present invention.
FIG. 11 is a sectional assembly view of the safety hypodermic syringe according to the second embodiment of the present invention.
FIG. 12 is a sectional view of the second embodiment of the present invention, showing the stopper moved with the plunger to the front limit position after injection.
FIG. 13 is a sectional view of the second embodiment of the present invention, showing the needle assembly and the separated tubular front neck carried to the inside of the barrel after the service of the safety hypodermic syringe.
FIG. 14 corresponds to FIG. 13, showing the needle cannula deformed.
FIG. 15 corresponds to FIG. 14, showing the plunger pulled backwards after deformation of the needle cannula.
FIG. 16 corresponds to FIG. 15, showing the shank separated from the head.

### DETAILED DESCRIPTION OF THE PREFERRED EMBODIMENT

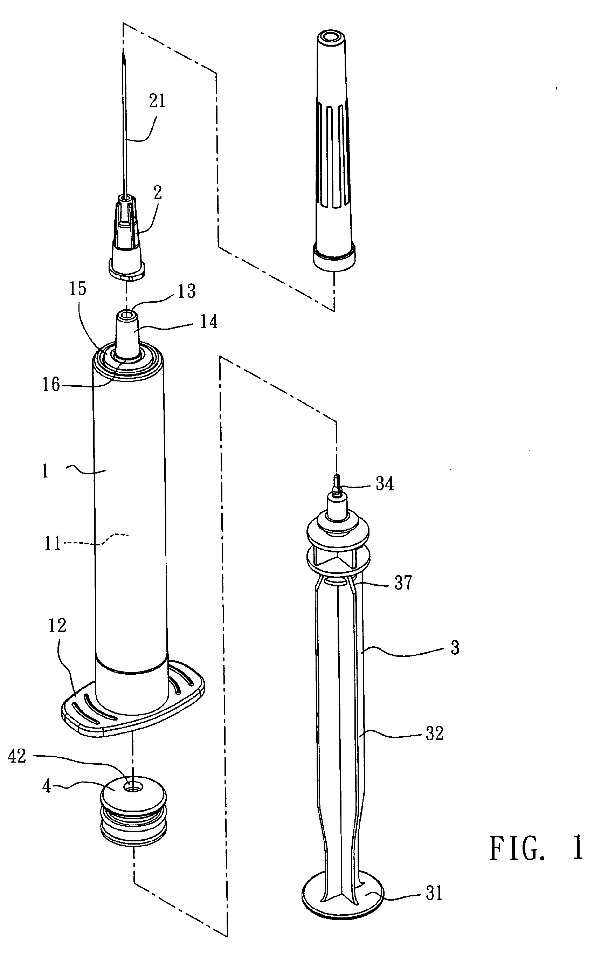

Referring to FIGS. 1∼3, a safety hypodermic syringe in accordance with a first embodiment of the present invention is shown comprised of a barrel **1,** a needle assembly **2,** a plunger **3,** and a stopper **4.**

Similar to the barrel of a conventional hypodermic syringe, the barrel **1** comprises a cylindrical body **11** adapted to hold a liquid medicine, a tubular neck **14** forwardly and axially extending from the front side of the cylindrical body **11** and defining a fluid passage **13** in communication between the inside space of the cylindrical body **11** and the atmosphere, and a finger flange **12** extending around the rear side of the cylindrical body **11.** Further, the barrel **1** has a front wall **15** that supports the tubular neck **14,** a breakable wall portion **16** formed in the front wall **15,** and an inside annular groove **17** extending around the inside wall near the finger flange **12.** The tubular neck **12** is formed integral with the front wall **15.** The breakable wall portion **16** is a thin wall portion that can be made in the shape of a straight line, spiral line, or S-line. Alternatively, two or more breakable wall portions **16** may be formed in the front wall **15.** The breakable wall portion **16** can easily be broken when the user pushes the plunger **3** with force, allowing the tubular neck **14** and the needle assembly **2** to be carried backwards into the inside of the cylindrical body **11** with the plunger **3** after the service of the hypodermic syringe.

The needle assembly **2** comprises a hub **22** and a needle cannula **21** fastened to the hub **22.** The hub **22** has a mounting hole **23** fitting the periphery of the tubular neck **14** and in fluid communication with the axial center through hole of the needle cannula **21.** Preferably, the tubular neck **14** and the mounting hole **23** are tapered.

The plunger **3** comprises a shank **32,** a thumb rest **31** at the rear side of the shank **32,** a head **33** at the front side of the shank **32** and adapted to hold the stopper **4** for allowing the stopper **4** to be moved along the inside wall of the cylindrical body **11** of the barrel 1 by the plunger **3,** and a tip **34** axially forwardly extending from the center of the front side of the head **33** and extending out of the front side of the stopper **4.** The tip **34** is barbed and insertable into the fluid passage **13** in the tubular neck **14** of the barrel **1.** In order to make the plunger breakable after the service of the safety hypodermic syringe, a reduced neck **37** is connected between the head **33** and the shank **32.** When biasing the plunger **3** against the inside wall of the barrel **1,** the neck **37** can easily be broken.

The stopper **4** is made of a flexible material, for example, rubber. The outer diameter of the stopper **4** fits the inner diameter of the cylindrical body **11** of the barrel **1** so that the stopper **4** seals the holding space of the cylindrical body **11** when inserted into the barrel **1.** The stopper **4** has a bottom coupling hole **41** coupled to the head **33** of the plunger **3,** and a front center through hole **42** forwardly extending from the bottom coupling hole **41** for the passing of the barbed tip **34.**

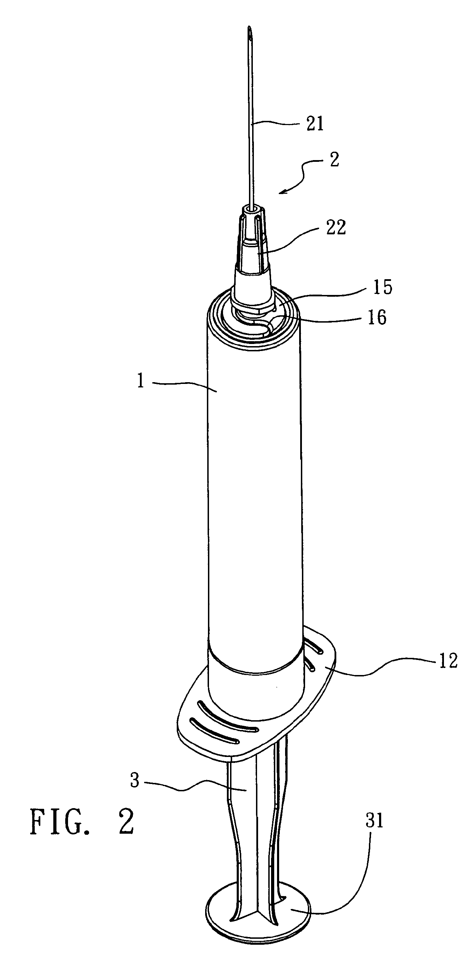

During the assembly process of the present invention, the stopper **4** is fastened to the head **33** of the plunger **3** to have the tip **34** extend out of the front center through hole **42,** and then the plunger **3** with the stopper **4** are inserted into the cylindrical body **11** of the barrel **1** from the rear side, and then the hub **21** of the needle assembly **2** is fastened to the tubular neck **14** of the barrel **1.** FIG. 2 shows the assembled status of the safety hypodermic syringe.

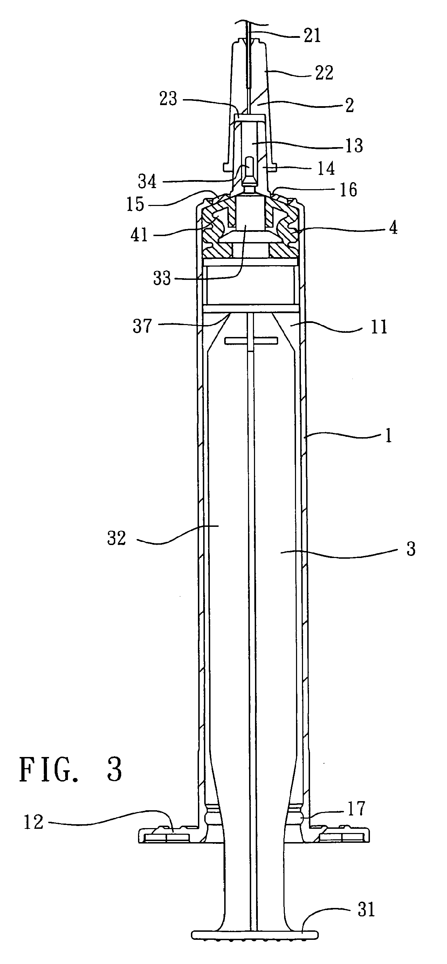

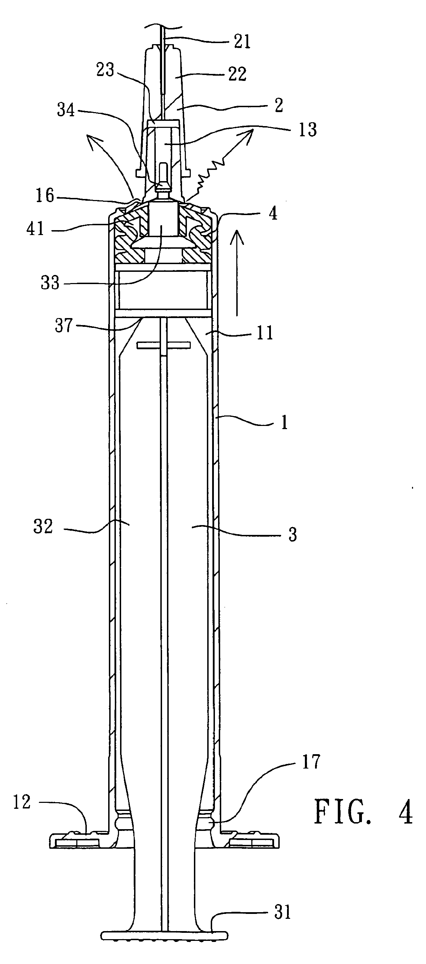

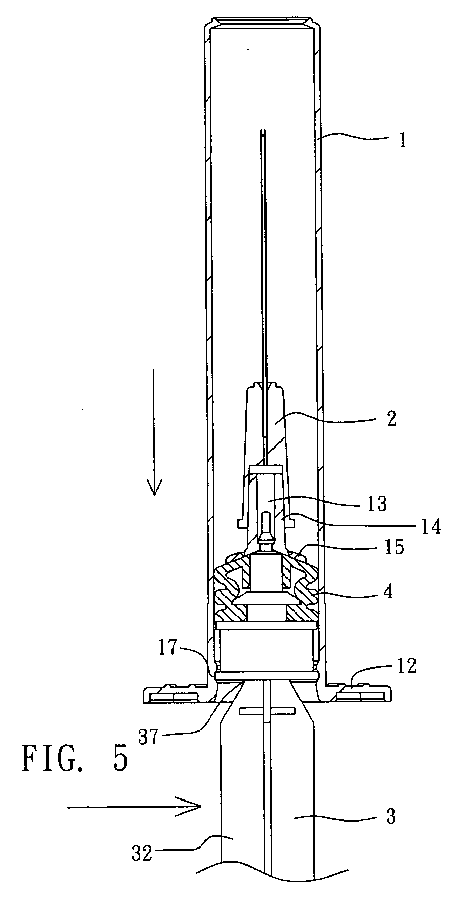

After the service of the safety hypodermic syringe as shown in FIG. 3, push the plunger **3** forwards again to force the barbed tip **34** into the tubular neck **14** and to impart a pressure to the front wall **15** of the barrel 1, thereby causing the breakable wall portion **16** to break as shown in FIG. 4. At this time, the broken barrel **1** becomes useless. When pulling the plunger **3** backwards at this time, the broken front wall **15** with the tubular neck **14** and the attached needle assembly **2** will be carried with the plunger **3** inside the cylindrical body **11** of the barrel **1,** as shown in FIG. 5.

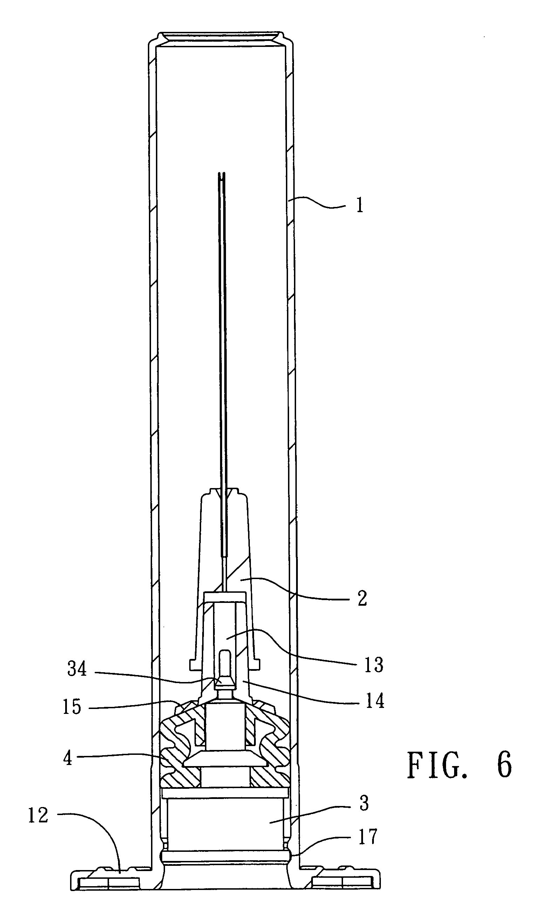

At this time, the user can bias the shank **32** against the inside wall of the cylindrical body **11** of the barrel **1** to break the neck **37,** as shown in FIG. 6.

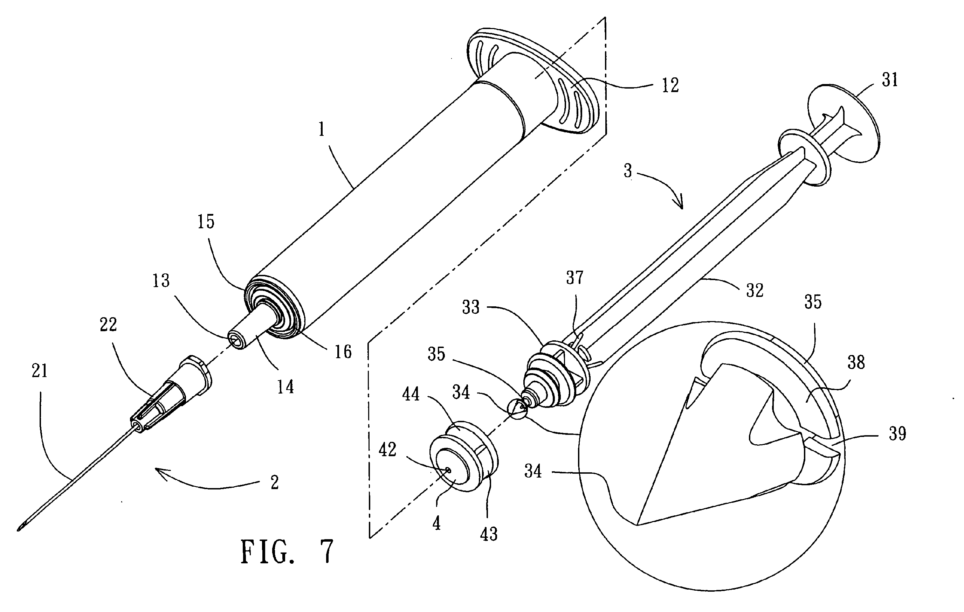

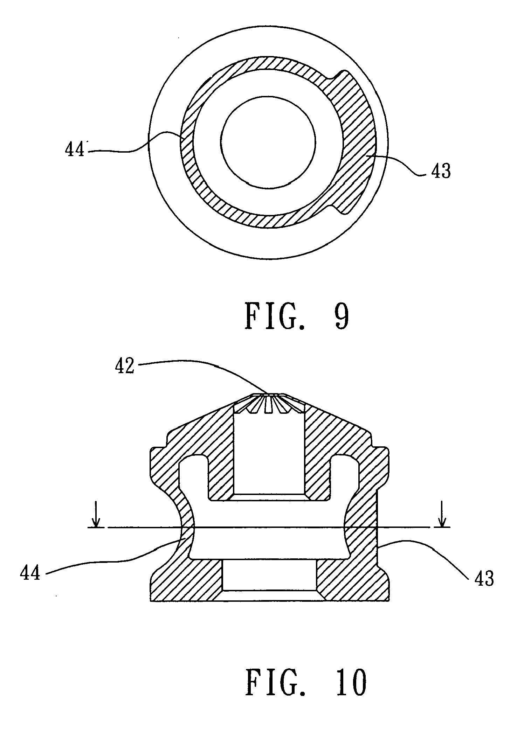

FIGS. 7~16 show a safety hypodermic syringe according to the second embodiment of the present invention. Similar to the aforesaid first embodiment, the safety hypodermic syringe according to this second embodiment is comprised of a barrel **1,** a needle assembly **2,** a plunger **3,** and a stopper **4.**

The barrel **1** is substantially similar to the aforesaid first embodiment with the exception of the tubular neck **14** has an inside annular flange **18** disposed near the top wall **15** of the barrel **1.**

The plunger **3** according to this second embodiment has a thin connecting portion **35** connected between the tip **34** and the head **33,** a split collar **38** extending around the connection area between the tip **34** and the thin connecting portion **35** and defining a step **36.** The split collar **38** has at least one radially extending crevice **39.** Further, the tip **34** according to this second embodiment is shaped like a conical arrowhead.

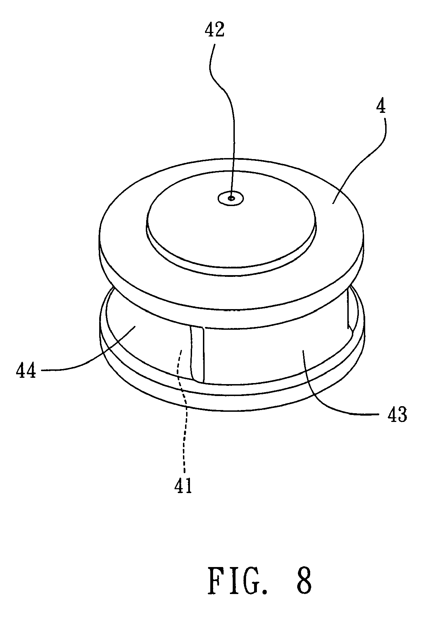

Referring to FIGS. 8~10, the peripheral wall of the stopper **4** has relatively thicker part forming a thick wall portion **43,** and a relatively thinner part forming a thin wall portion **44.** The peripheral wall of the stopper **4** can be made smoothly curved inwards at different extents at different locations, forming the thick wall portion **43** and the thin wall portion **44.**

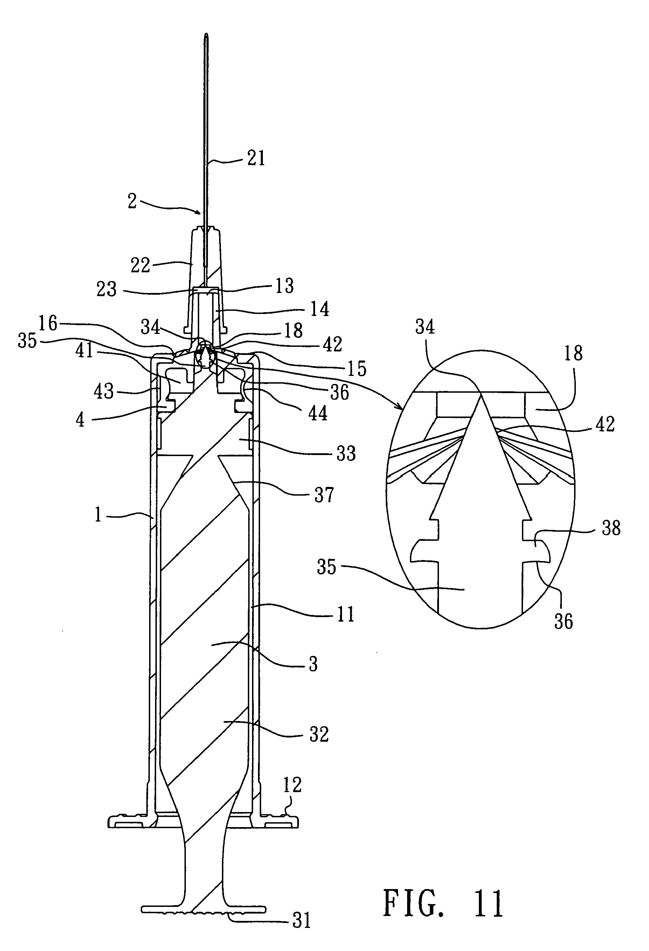

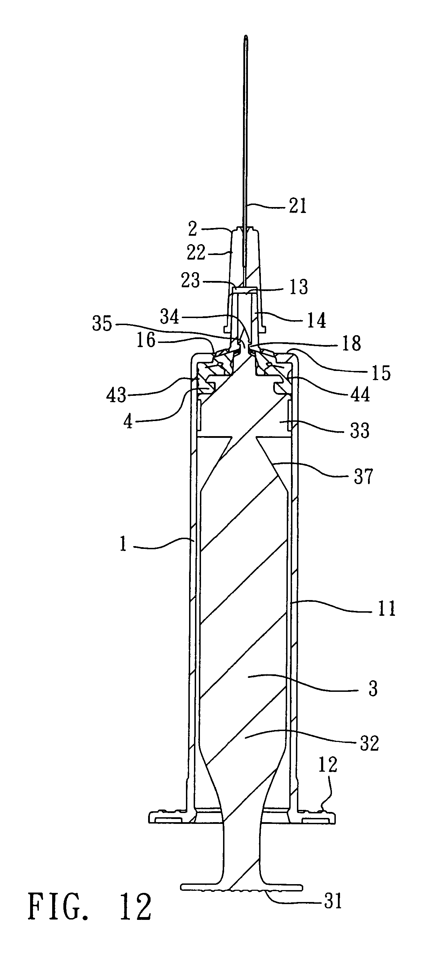

FIG. 11 shows the assembled status of the hypodermic syringe of this second embodiment. When pushed the plunger **3** to the front side to force the liquid medicine into the patient's body as shown in FIG. 12, the tip **34** and the split collar **38** are forced out of the front center through hole **42** of the stopper **4** and the stopper **4** is stopped against the front wall **15** of the barrel **1** to squeeze residual liquid medicine out of the tubular neck **14** of the barrel **1**, and the step **36** is stopped between the front wall **15** of the barrel **1** and the inside annular flange **18.**

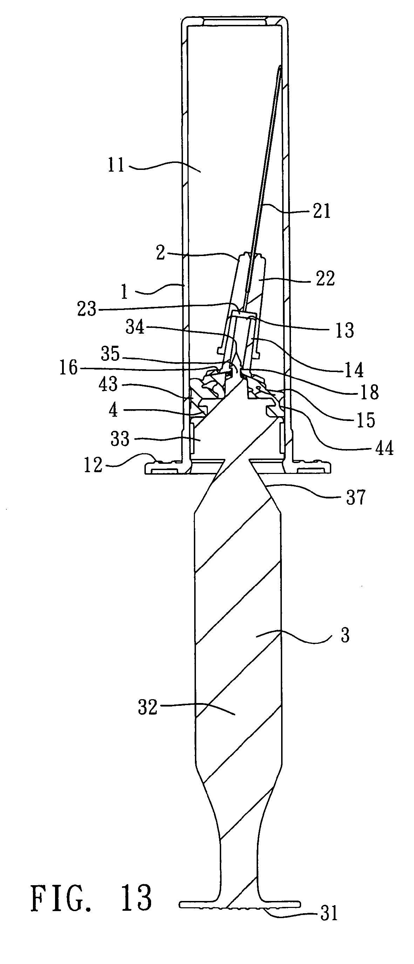

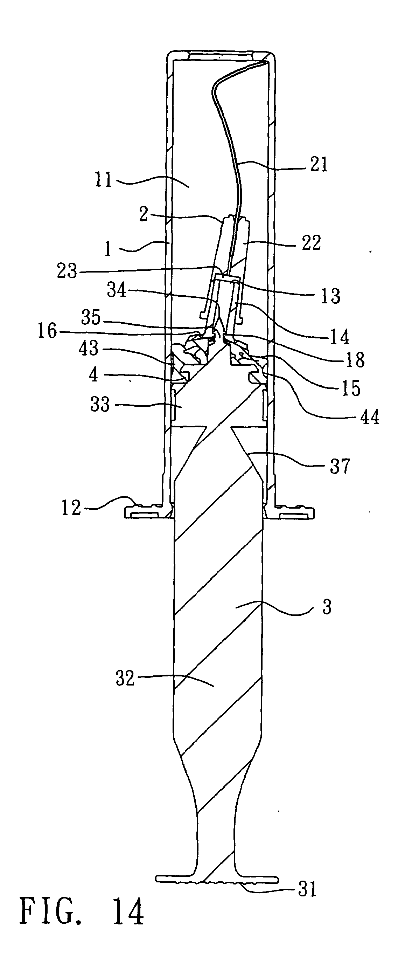

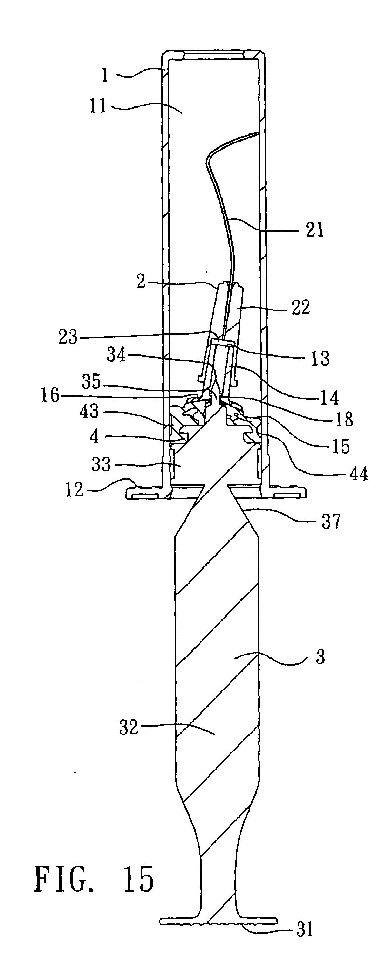

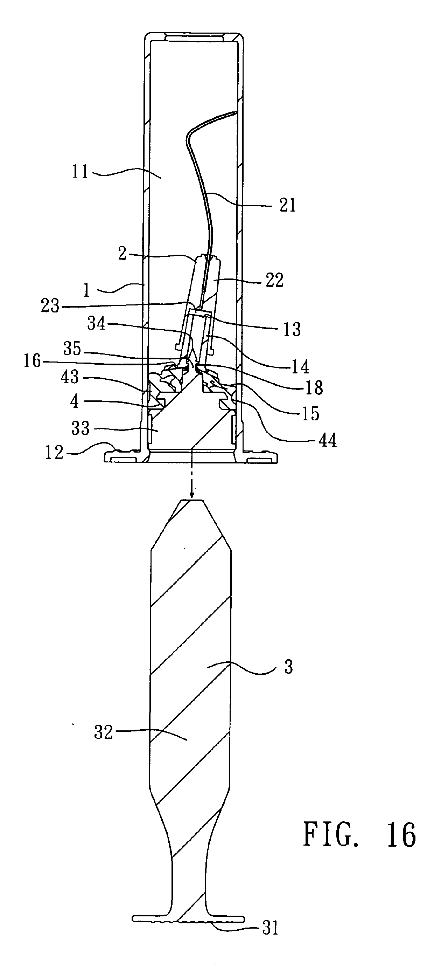

When pulling the plunger **3** backwards after injection, the breakable wall portion **16** will be broken and the needle assembly **2** will be carried into the inside of the cylindrical body **11** of the barrel **1.** Because the stopper **4** is elastic and has a thick wall portion **43** and a thin wall portion **44,** the stopper **4** will be deformed and forced to bias the needle assembly 2 toward the thin wall portion **44** during backward movement of the needle assembly **2** with the plunger **3,** as shown in FIG. 13. When pushing the plunger **3** forwards at this time, the needle cannula **21** will be stopped against the inside wall of the barrel **1** and permanently deformed as shown in FIG. 14. At this time, the user can break the neck **37** (see FIG. 15) and separate the shank **33** from the head **33** (see FIG. 16). Thus, the waste hypodermic syringe can be disposed of safely.

Simply forming a breakable wall portion on the front wall of the barrel, the hypodermic syringe becomes self-destructive. This simple structural design saves much manufacturing cost. The safety hypodermic syringe of the second embodiment of the present invention provides the self-destructive function and can completely force liquid medicine out of the barrel, preventing residual liquid medicine in the barrel.

A prototype of safety hypodermic syringe has been constructed with the features of FIGS. 1~16. The safety hypodermic syringe functions smoothly to provide all the features discussed earlier.

Although a particular embodiment of the invention has been described in detail for purposes of illustration, various modifications and enhancements may be made without departing from the spirit and scope of the invention. Accordingly, the invention is not to be limited except as by the appended claims.

## Claims

1. A safety hypodermic syringe comprising:
a barrel, said barrel comprising a cylindrical body, said cylindrical body having a front wall, and a tubular front neck forwardly extending from said front wall, the front wall of said cylindrical body having a thin wall portion that forms at least one breakable wall portion through which the front wall of said cylindrical body is breakable for enabling said tubular front neck to be separated from said cylindrical body;
a plunger axially movably inserted into said cylindrical body of said barrel, said plunger comprising a shank, a head formed integral with a front end of said shank, and a barbed tip forwardly extending from said head; and
a flexible stopper mounted on the head of said plunger and peripherally maintained in close contact with the periphery of said cylindrical body of said barrel and axially movable with said plunger relative to said barrel, said flexible stopper having a front center through hole through which said barbed tip of said plunger extends to the outside of said flexible stopper for engaging into the fluid passage of said tubular front neck of said barrel when said plunger is pushed forward and forced against said front wall of said cylindrical body of said barrel to break said at least one breakable wall portion and to separate said tubular front neck from said cylindrical body for enabling said needle assembly to be carried with the separated tubular front neck by said plunger into the inside of said barrel after the service of the safety hypodermic syringe.

2. The safety hypodermic syringe as claimed in claim 1, wherein said barrel has a finger flange at a rear side thereof, and an inside annular groove extending around the inside wall thereof near said finger flange; said plunger has a thumb rest at a rear side thereof.

3. The safety hypodermic syringe as claimed in claim 1, wherein said plunger has a breakable neck connected between said head and said shank.

4. The safety hypodermic syringe as claimed in claim 1, wherein the number of said at least one thin breakable wall portion of said front wall of said barrel is 2, and each of said at least one breakable wall portion has a S-shape.

5. A safety hypodermic syringe comprising:
a barrel, said barrel comprising a cylindrical body, said cylindrical body having a front wall, and a tubular front neck forwardly extending from said front wall, the front wall of said cylindrical body having a thin wall portion that forms at least one breakable wall portion through which the front wall of said cylindrical body is breakable for enabling said tubular front neck to be separated from said cylindrical body, said tubular front neck having an inside annular flange;
a needle assembly, said needle assembly comprising a hub fastened to said tubular front neck of said barrel and defining a fluid passage, and a needle cannula fastened to said hub;
a plunger axially movably inserted into said cylindrical body of said barrel, said plunger comprising a shank and a head formed integral with a front end of said shank; and
a flexible stopper mounted on a front side of the head of said plunger and peripherally maintained in close contact with the periphery of said cylindrical body of said barrel and axially movable with said plunger relative to said barrel, said flexible stopper having a thin wall portion and a thick wall portion disposed at two opposite lateral sides;
wherein when said plunger is pushed forwards and forced against said front wall of said cylindrical body of said barrel, said at least one breakable wall portion is broken to separate said tubular front neck of said barrel from said cylindrical body for enabling said needle assembly to be carried with the separated tubular front neck by said plunger into the inside of said barrel after the service of the safety hypodermic syringe, and at the same time said stopper is deformed to tilt said needle assembly inside said cylindrical body.

6. The safety hypodermic syringe as claimed in claim 5, wherein said stopper has a part of a peripheral wall thereof smoothly curved inwards at different extents, forming said thin wall portion and said thick wall portion.

7. The safety hypodermic syringe as claimed in claim 5, wherein said stopper has a front through hole; said plunger has an arrowhead-like front tip insertable through the front through hole of said stopper into said tubular front neck of said barrel when said stopper is pushed forwards by said plunger and stopped against the front wall of said cylindrical body of said barrel.

8. The safety hypodermic syringe as claimed in claim 5, wherein said plunger has a thin breakable connecting portion connected between said head and said arrowhead-like tip; said barrel has a finger flange at a rear side thereof.

9. A safety hypodermic syringe comprising:
a barrel, said barrel comprising a cylindrical body, said cylindrical body having a front wall, and a tubular front neck forwardly extending from said front wall of said cylindrical body and defining a fluid passage;
a needle assembly, said needle assembly comprising a hub fastened to said tubular front neck of said barrel and defining a fluid passage, and a needle cannula fastened to said hub;
a plunger axially movably inserted into said cylindrical body of said barrel, said plunger comprising a shank, a head formed integral with a front end of said shank, and a front tip forwardly extending from said head; and
a flexible stopper mounted on the head of said plunger over said front tip and peripherally maintained in close contact with the periphery of said cylindrical body of said barrel and axially movable with said plunger relative to said barrel, said flexible stopper having a front center through hole through which said front tip of said plunger is moved into the inside of said tubular front neck of said barrel to expel residual liquid medicine out of said barrel when said stopper is forced by said plunger against the front wall of said cylindrical body of said barrel during the application of the safety hypodermic syringe.

10. The safety hypodermic syringe as claimed in claim 9, wherein said the front wall of said cylindrical body of said barrel has a thin portion forming at least one breakable wall portion; said tubular front neck of said barrel has an inside annular flange; said plunger has a split collar spaced between said head and said barbed tip for engaging with the inside annular flange of said tubular front neck after the operation of the safety hypodermic syringe for enabling said tubular front neck to be separated from the front wall of said cylindrical body of said barrel and moved with said needle assembly into the inside of said by said plunger after said plunger having pushed said stopper against the front wall of said cylindrical body of said barrel to break said at least one breakable wall portion.

11. The safety hypodermic syringe as claimed in claim 10, wherein said stopper has a thin wall portion and a thick wall portion disposed at two opposite sides for enabling said stopper to tilt said needle assembly when said needle assembly is carried with said tubular front neck into the inside of said cylindrical body of said barrel by said plunger.

12. The safety hypodermis syringe as claimed in claim 10, wherein said plunger comprises a breakable neck connected between said head and said shank, and a thumb rest at a rear side of said shank remote from said head.
